(19) Europäisches Patentamt
European Patent Office
Office européen des brevets

(11) Veröffentlichungsnummer: **0 004 642**
**A1**

# (12) EUROPÄISCHE PATENTANMELDUNG

(21) Anmeldenummer: 79100944.2

(22) Anmeldetag: 29.03.79

(51) Int. Cl.[2]: C 07 C 87/60
C 07 D 319/08, C 07 C 93/14
C 07 C 121/78, C 07 C 147/12
C 07 C 149/42, A 01 N 9/20
A 01 N 9/12, A 01 N 9/14

(30) Priorität: 08.04.78 DE 2815290

(43) Veröffentlichungstag der Anmeldung: 17.10.79 Patentblatt 79/21

(84) Benannte Vertragsstaaten: BE CH DE FR GB IT NL

(71) Anmelder: BAYER Aktiengesellschaft
Zentralbereich Patente,Marken und Lizenzen Bayerwerk
D-5090 Leverkusen 1(DE)

(72) Erfinder: Hartmann, Alfons, Dr.
Poststrasse 35
D-6645 Beckingen 4(DE)

(72) Erfinder: Klauke, Erich, Dr.
Eichendorffweg 8
D-5068 Odenthal(DE)

(72) Erfinder: Behrenz, Wolfgang, Dr.
Untergruendemich 14
D-5063 Overath(DE)

(72) Erfinder: Frohberger, Paul-Ernst, Dr.
Willi-Baumeister-Strasse 5
D-5090 Leverkusen(DE)

(72) Erfinder: Hammann, Ingeborg, Dr.
Belfortstrasse 9
D-5000 Köln(DE)

(72) Erfinder: Homeyer, Bernhard, Dr.
Obere Strasse 28
D-5090 Leverkusen 3(DE)

## (54) Neue Diarylamine, Verfahren zu ihrer Herstellung und ihre Verwendung.

(57) Es wurden die Diarylamine der allgemeinen Formel I gefunden,

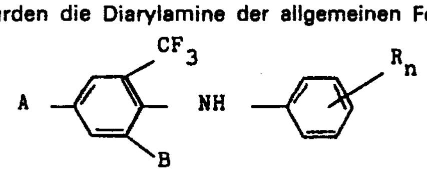

(I)

in welcher
A und B voneinander verschieden sind und für Trifluormethyl oder Nitro stehen,
n für eine ganze Zahl von 1 bis 5 steht und
R für gleiche oder verschiedene Reste aus der Gruppe Wasserstoff, Halogen, Cyan, Nitro, Alkyl, Halogenalkyl, Alkenyl, Alkinyl, Alkoxy, Alkylmercapto, Alkylsulfonyl, Halogenalkoxy, Halogenalkylmercapto, Halogenalkylsulfonyl, Aryloxy, Arylmercapto steht,
wobei ortho-ständige Substituenten R gemeinsam mit den beiden angrenzenden C-Atomen des Phenylkerns einen gegebenenfalls halogensubstituierten Heterocyclus bilden können,
und einschränkend gilt, dass $R_n$ nicht 2,4,6-Trinitro, 2,6-Dinitro-4-cyan, 2,6-Dinitro-4-trifluormethyl oder 2,4-Dinitro-6-trifluormethyl bedeutet.

Die Diarylamine werden erhalten, wenn man
a) eine Verbindung der Formel (II)

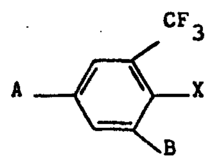

(II)

in welcher
X für Fluor, Chlor oder Brom steht mit einem Anilin umsetzt, oder
b) ein Anilin der Formel (IV)

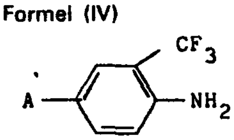

(IV)

in welcher
A und B die oben angegebene Bedeutung haben, mit einer Verbindung der Formel (V)

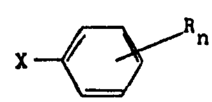

(V)

umsetzt.

Die Diarylamine der Formel zeigen eine insektizide, akarizide, nematizide und fungizide Wirkung.

Diese Verbindungen enthaltende Zusammensetzungen und ihre Verwendung sind beschrieben.

EP 0 004 642 A1

BAYER AKTIENGESELLSCHAFT
Zentralbereich
Patente, Marken und Lizenzen

5090 Leverkusen, Bayerwerk
Rt/AB
Typ Ib

Neue Diarylamine, Verfahren zu ihrer Herstellung und ihre Verwendung

Die vorliegende Erfindung betrifft neue Diarylamine, Verfahren zu ihrer Herstellung sowie ihre Verwendung als Schädlingsbekämpfungsmittel.

Es sind bereits Diarylamine mit insektiziden, akariziden und fungiziden Eigenschaften bekannt geworden (vgl. Deutsche Offenlegungsschrift 2 509 416). Ihre Wirkung ist jedoch, vor allem bei niedrigen Aufwandkonzentrationen, nicht voll befriedigend.

Es wurden die neuen Diarylamine der allgemeinen Formel (I) gefunden,

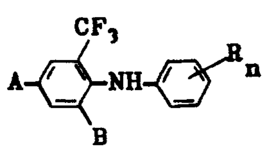

(I)

in welcher

A und B voneinander verschieden sind und für Trifluormethyl oder Nitro stehen,
n für eine ganze Zahl von 1 bis 5 steht und
R für gleiche oder verschiedene Reste aus der Gruppe Wasserstoff, Halogen, Cyan, Nitro, Alkyl, Halogenalkyl, Alkenyl, Alkinyl, Alkoxy, Alkylmercapto, Alkylsulfonyl, Halogenalkoxy, Halogenalkylmercapto, Halogenalkylsulfonyl, Aryloxy, Arylmercapto steht,
wobei ortho-ständige Substituenten R gemeinsam mit den beiden angrenzenden C-Atomen des Phenylkerns einen gegebenenfalls halogensubstituierten Heterocyclus bilden können,
und einschränkend gilt, daß $R_n$ nicht 2,4,6-Trinitro, 2,6-Dinitro-4-cyan, 2,6-Dinitro-4-trifluormethyl oder 2,4-Dinitro-6-trifluormethyl bedeutet.
Diese Verbindungen weisen ausgezeichnete insektizide, akarizide, nematizide und fungizide Eigenschaften auf.

Weiterhin wurde gefunden, daß man die neuen Diarylamine der Formel (I) erhält, wenn man

a) eine Verbindung der Formel (II)

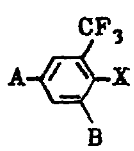 (II)

in welcher
X für Fluor, Chlor oder Brom steht und
A und B die oben angegebene Bedeutung haben,

mit einem Anilin der Formel (III)

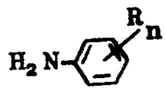 (III)

in welcher
R und n die oben angegebene Bedeutung haben,
in Gegenwart eines säurebindenden Mittels sowie gegebenenfalls in Gegenwart eines Verdünnungsmittels umsetzt, oder

b) ein Anilin der Formel (IV)

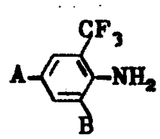 (IV)

in welcher
A und B die oben angegebene Bedeutung haben,

mit einer Verbindung der Formel (V)

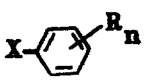 (V)

in welcher
X für Fluor, Chlor oder Brom steht und
R und n die oben angegebene Bedeutung haben,

in Gegenwart eines säurebindenden Mittels sowie gegebenenfalls in Gegenwart eines Verdünnungsmittels umsetzt.

Überraschenderweise zeigen die neuen Diarylamine der Formel (I) eine wesentlich höhere insektizide, akarizide,

nematizide und fungizide Potenz als die bekannten Diarylamine. Die erfindungsgemäßen Stoffe stellen somit eine Bereicherung der Technik dar.

Bevorzugt sind Verbindungen der Formel I in der R für $C_{1-4}$ Alkylsulfonyl, Halogen insbesondere Fluor, Chlor oder Brom, Trifluormethyl, Cyan, Nitro, Trifluormethoxy, Trifluormethylmercapto, Trifluormethylsulfonyl, gegebenenfalls chlorsubstituiertes Phenoxy oder Phenylmercapto steht oder 2 ortho-ständige Reste R gemeinsam mit den angrenzenden C-Atomen einen fluorsubstituierten Dioxanylring bilden und n für 1, 2 oder 3 steht.

Besonders bevorzugt sind Verbindungen der Formel (I), in welcher R für $C_1$-$C_4$-Alkylsulfonyl, Halogen, insbesondere Chlor, Trifluormethyl, Cyan, Nitro, Trifluormethoxy, Trifluormethylmercapto, Trifluormethylsulfonyl und n für 1, 2 oder 3 steht.

Im einzelnen seien folgende Verbindungen der Formel I genannt:

2,4,6-Trichlor-4'-nitro-2',6'-bis-trifluormethyl-diphenylamin,
2,6-Dichlor-4'-nitro-4,2',6'-tris-trifluormethyl-diphenylamin,
2,6-Dichlor-2'-nitro-4,4',6'-tris-trifluormethyl-diphenylamin,
4-Nitro-2,6-bis-trifluormethyl-4'-trifluormethylsulfonyl-diphenylamin,
2-Chlor-4'-nitro-2',6'-bis-trifluormethyl-4-trifluormethylsulfonyl-diphenylamin.

Verwendet man 2-Chlor-5-nitro-1,3-bis-trifluormethylbenzol und 2,6-Dichlor-4-trifluormethylanilin als Ausgangsstoffe, so läßt sich der Reaktionsablauf durch das folgende Formelschema wiedergeben:

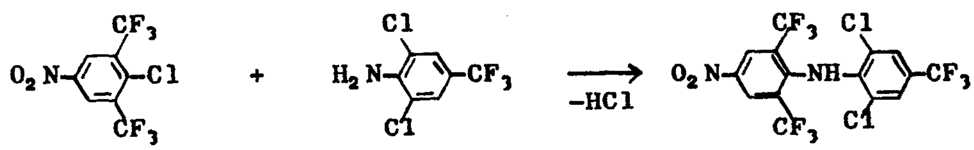


**Die als Ausgangsstoffe zu verwendenden Verbindungen II, III, IV und V sind bekannt oder lassen sich nach bekannten Verfahren herstellen.**

**Vorzugsweise werden die neuen Wirkstoffe durch Umsetzung** von 2-Chlor-5-nitro-1,3-bis-trifluormethylbenzol oder 2-Chlor-3-nitro-1,5-bis-trifluormethylbenzol mit einem Anilin der Formel (III) hergestellt, in welcher **R für gleiche oder verschiedene Reste aus der Gruppe Wasserstoff, Halogen, Cyan, Nitro, Alkyl, Alkoxy, Alkylmercapto, Trifluormethylsulfonyl, Alkylsulfonyl, ggf. durch Chlor oder Trifluormethyl substituiertes Phenoxy oder Phenylmercapto und n für 1,2 oder 3 steht. Dabei können ortho-ständige Substituenten R gemeinsam mit den beiden angrenzenden C-Atomen des Phenylkerns einen gegebenenfalls halogensubstituierten Dioxanylring bilden. Als Beispiele für erfindungsgemäß verwendbare Aniline der Formel (III) seien genannt:**

**2,3-Dichlor-anilin**
**2,4-Dichlor-anilin**

2,5-Dichlor-anilin
2,6-Dichlor-anilin
3,4-Dichlor-anilin
2,4,6-Trichlor-anilin
2,4,5-Trichlor-anilin
3,4,5-Trichlor-anilin
2,4-Difluor-anilin
3-Chlor-4-fluor-anilin
3-Chlor-4-nitro-anilin
2,6-Dichlor-4-nitro-anilin
2-Brom-4-nitro-anilin
2-Methoxy-4-nitro-anilin
2,4-Dinitro-anilin
2-Chlor-6-methyl-anilin
2,6-Dichlor-4-trifluormethyl-anilin
2-Chlor-5-trifluormethyl-anilin
3-Chlor-4-trifluormethyl-anilin
4-Chlor-3-trifluormethyl-anilin
4-Chlor-2-trifluormethyl-anilin
2,4-Bis-trifluormethyl-anilin
3,5-Bis-trifluormethyl-anilin
4-Nitro-2-trifluormethyl-anilin
4-Methylsulfonyl-2-trifluormethyl-anilin
2-Ethylsulfonyl-5-trifluormethyl-anilin
2-Amino-5-trifluormethyl-benzonitril
2-Amino-5-chlor-benzonitril
2-Amino-4-nitro-benzonitril
2,5-Dichlor-4-amino-benzonitril
4-Amino-phthalsäuredinitril
4-Amino-isophthalsäuredinitril
4-Trifluormethoxy-anilin
3-Chlor-4-trifluormethylmercapto-anilin

2-Chlor-5-trifluormethylmercapto-anilin
4-Trifluormethylsulfenyl-anilin
2-Chlor-4-trifluormethylsulfonyl-anilin
2,6-Dichlor-4-trifluormethylsulfonyl-anilin
2-(4-Chlorphenylthio)-5-trifluormethyl-anilin
4-(4-Chlorphenoxy)-3-trifluormethyl-anilin
4-(2-Chlor-5-trifluormethyl-phenoxy)-anilin
6-Amino-2,2,4,4-tetrafluor-1,3-benzodioxen

Als Verdünnungsmittel eignen sich inerte organische Lösungsmittel. Bevorzugt werden Dimethylformamid oder Tetrahydrofuran verwendet.

Als säurebindende Mittel eignen sich Basen wie Alkalimetallhydroxide, -carbonate oder -hydride. Bevorzugt verwendet man Kaliumhydroxid oder Natriumhydrid.

Die Reaktionstemperatur kann in einem größeren Bereich variiert werden. Im allgemeinen arbeitet man zwischen -10 und + 100°, vorzugsweise bei 0 bis 40°.

Gewöhnlich werden die Reaktionspartner in äquimolaren Mengen eingesetzt, doch auch die Verwendung der einen oder anderen Komponente im Ueberschuß ist möglich.

Die erfindungsgemäßen Verbindungen besitzen neben ihrer insektiziden,akariziden und nematiziden Wirkung auch fungizide Wirkungen gegen Schorf, Rost und Mehltaupilze insbesondere gegen Pyricularia und Pellicularia-Arten. Besonders geeignet sind sie zur fungiziden Verwendung in Saatgutbeizmitteln und Sproßbehandlungsmitteln gegen Getreidekrankheiten.

Einige der erfindungsgemäßen Verbindungen zeigen vor allem bei höherem Aufwandkonzentrationen herbizide Wirkung.

Die Wirkstoffe eignen sich zur Bekämpfung von tierischen Schädlingen, insbesondere Insekten, Spinnentieren und Nematoden, die in der Landwirtschaft, in Forsten, im Vorrats- und Materialschutz sowie auf dem Hyginesektor vorkommen. Sie sind gegen normal sensible und resistente Arten sowie gegen alle oder einezlne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria  migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

$LT_{100}$-Test für Dipteren

| | |
|---|---|
| Testtiere: | Aedes aegyptii |
| Zahl der Testtiere: | 20 |
| Lösungsmittel: | Aceton |

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 3, 2, 4, 24, 9, 38.

Beispiel B

$LD_{100}$-Test

Testtiere: Blatta orientalis
Zahl der Testtiere: 10
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 31, 3, 28, 4, 2.

Beispiel C

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 21, 31, 41, 47.

Beispiel D

Tetranychus-Test (resistent)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 13, 4, 3, 7, 14, 38.

Beispiel E

Plutella-Test

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 13, 4, 43, 3, 7, 14, 38.

Beispiel F

Grenzkonzentrations-Test / Bodeninsekten

Testinsekt: Phrobia antiqua Maden (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm ( = mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 4, 2, 9, 13, 14, 21, 25, 3, 31, 1, 38, 43.

Beispiel G

Grenzkonzentrations-Test / Bodeninsekten

| | | | |
|---|---|---|---|
| Testinsekt: | Tenebrio molitor Larven (im Boden) | | |
| Lösungsmittel: | 3 | Gewichtsteile | Aceton |
| Emulgator: | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden,welche in ppm ( = mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 4, 2, 9, 13, 14, 15, 16, 17.

Beispiel H

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 4, 1, 38.

Beispiel I

Sproßbehandlungs-Test / Getreiderost / protektiv (blattzerstörende Mykose)

Zur Herstellung einer zweckmäßigen Wirkstoffzubereitung nimmt man 0,25 Gew.-Teile Wirkstoff in 25 Gew.-Teilen Dimethylformamid und 0,06 Gew.-Teilen Emulgator Alkylaryl-polyglykoläther auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia reconditia in 0,1 %igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100 %igen Luftfeuchtigkeit in ein Gewächshaus.

Nach 10 Tagen Verweilzeit der Pflanzen bei einer Temperatur von 20°C und einer Luftfeuchtigkeit von 80 - 90 % wertet man den Besatz der Pflanzen mit Rostpusteln aus. Der Befallgrad wird in Prozenten des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Rostbefall ist.

Bei diesem Test zeigen z.B. die folgenden Verbindungen der Herstellungsbeispiele überlegene Wirksamkeit gegenüber dem Stand der Technik: 2, 4, 5, 8, 11, 19, 3, 32, 33, 34, 37, 38.

Beispiel K

Sproßbehandlungs-Test / Getreidemehltau / protektiv (blattzerstörende Mykose)

Zur Herstellung einer zweckmäßigen Wirkstoffzubereitung nimmt man 0,25 Gew.-Teile Wirkstoff in 25 Gewichtsteilen Dimethylformamid und 0,06 Gew.-Teilen Emulgator (Alkyl-aryl-polyglykoläther) auf und gibt 975 Gew.-Teile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 - 22°C und einer Luftfeuchtigkeit von 80 - 90 % wertet man den Besatz der Pflanzen mit Mehltaupusteln aus. Der Befallsgrad wird in Prozenten des Befalls der unbehandelten Kontrollpflanzen ausgedrückt. Dabei bedeutet 0 % keinen Befall und 100 % den gleichen Befallsgrad wie bei der unbehandelten Kontrolle. Der

Wirkstoff ist umso wirksamer, je geringer der Mehltaubefall ist.

Bei diesem Test zeigen z.B. die folgenden Verbindungen der Herstellungsbeispiele überlegene Wirksamkeit gegenüber dem Stand der Technik: 19, 3, 38.

Beispiel L

Saatgutbeizmittel-Test / Weizensteinbrand
(samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Man kontaminiert Weizensaatgut mit 5 g Chlamydosporen von Tilletia caries pro kg Saatgut. Zur Beizung schüttelt man das Saatgut mit dem Beizmittel in einer verschlossenen Glasflasche. Das Saatgut wird auf feuchtem Lehm unter einer Deckschicht aus einer Lage Mull und 2 cm mäßig feuchter Komposterde 10 Tage lang im Kühlschrank bei 10°C optimalen Keimungsbedingungen für die Sporen ausgesetzt.

Anschließend bestimmt man mikroskopisch die Keimung der Sporen auf den Weizenkörnern, die jeweils mit rund 100 000 Sporen besetzt sind. Der Wirkstoff ist umso wirksamer je weniger Sporen gekeimt sind.

Bei diesem Test zeigen z.B. die folgenden Verbindungen der Herstellungsbeispiele überlegene Wirksamkeit gegenüber dem Stand der Technik: 4, 2.

Herstellungsbeispiele

1.

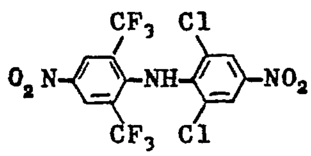

Eine Lösung von 10,4g(50 mMol) 2,6-Dichlor-4-nitro-anilin in 100 ml trockenem Dimethylformamid wird bei Raumtemperatur portionsweise mit 5,6 g (100 mMol) pulverisiertem Kaliumhydroxid versetzt. Anschließend wird bei 25° eine Lösung von 14,7g(50 mMol) 1-Chlor-4-nitro-2,6-bis-trifluormethyl-benzol in 80 ml trockenem Dimethylformamid zugetropft. Man rührt über Nacht bei Raumtemperatur, versetzt mit 100 ml Eisessig, gießt auf Eis und saugt 22,5 g (97 % d. Th.) 2,6-Dichlor-4,4'-dinitro-2',6'-bis-trifluormethyl-diphenylamin ab. Fp. 190°.

2.

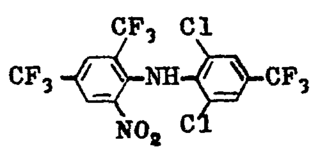

Zu einer Suspension von 40,6 g 80proz. Natriumhydrid (20 % Paraffin) in 750 ml trockenem Dimethylformamid tropft man bei 0° eine Lösung von 161 g (0,7 Mol) 2,6-Dichlor-4-trifluormethyl-anilin in 200 ml Dimethylformamid. Man rührt eine Stunde bei Raumtemperatur und tropft dann bei 0° eine Lösung von 205 g (0,7 Mol) 1-Chlor-2-nitro-4,6-bis-trifluormethyl-benzol in 200 ml

Dimethylformamid zu. Man beläßt 3 Tage bei Raumtemperatur, gießt auf ein Gemisch von 700 ml konz. Salzsäure und 1 kg Eis und verdünnt mit Wasser auf ein Volumen von 10 ltr. Das zunächst ölig abgeschiedene Produkt kristallisiert nach etwa 3 - 4 Stunden. Es wird abgesaugt und mit Wasser und Petroläther gewaschen. Fp. 88°, Ausbeute 76 % d. Th.

3.

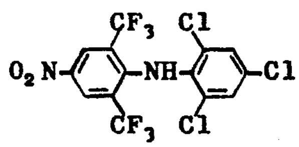

Zu einer Lösung von 3,93 g (0,02 Mol) 2,4,6-Trichlor-anilin in 50 ml trockenem Dimethylformamid gibt man 1,2 g 80proz. Natriumhydrid (20 % Paraffin). Man rührt 1 Stunde bei Raumtemperatur, kühlt auf 10° ab und tropft bei dieser Temperatur eine Lösung von 5,87 g (0,02 Mol) 1-Chlor-4-nitro-2,6-bis-trifluormethyl-benzol in 50 ml trockenem Dimethylformamid zu. Nach Stehen über Nacht wird mit 100 ml Eisessig versetzt und auf Eis gegossen. Durch Absaugen erhält man 7,43 g (82 % d. Th.) 2,4,6-Trichlor-4'-nitro-2',6'-bis-trifluormethyl-diphenylamin, Fp. 115°.

Analog Beispiel 3 wurden die folgenden Verbindungen erhalten:

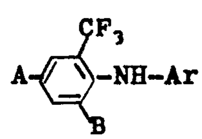

| Beispiel Nr. | A | B | Ar | Fp ($^{o}C$) |
|---|---|---|---|---|
| 4 | $NO_2$ | $CF_3$ | 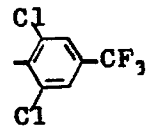 | 89 |
| 5 | $NO_2$ | $CF_3$ | 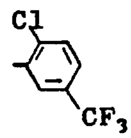 | 85 - 86 |
| 6 | $CF_3$ | $NO_2$ | 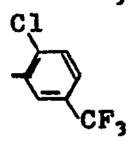 | 97 |
| 7 | $NO_2$ | $CF_3$ | 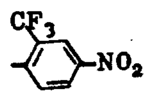 | 135 |
| 8 | $NO_2$ | $CF_3$ | 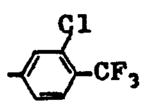 | 111 |
| 9 | $CF_3$ | $NO_2$ | 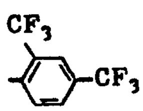 | 74 |
| 10 | $CF_3$ | $NO_2$ | 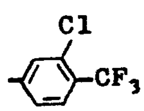 | 96 |
| 11 | $NO_2$ | $CF_3$ | 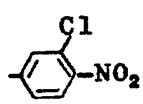 | 98 - 100 |
| 12 | $CF_3$ | $NO_2$ | 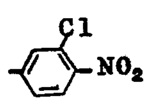 | 164 |
| 13 | $NO_2$ | $CF_3$ | 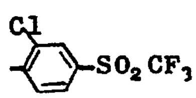 | 138 |
| 14 | $CF_3$ | $NO_2$ | 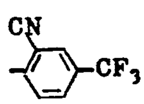 | 95 |

| | | | | |
|---|---|---|---|---|
| 15 | $CF_3$ | $NO_2$ | 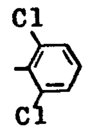 | 95 |
| 16 | $NO_2$ | $CF_3$ | 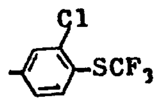 | 90 |
| 17 | $NO_2$ | $CF_3$ | 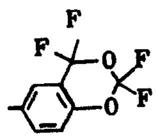 | 102 |
| 18 | $CF_3$ | $NO_2$ | 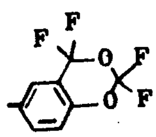 | 92 - 93 |
| 19 | $CF_3$ | $NO_2$ | 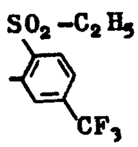 | 103 |
| 20 | $NO_2$ | $CF_3$ | 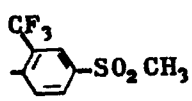 | 198 |
| 21 | $NO_2$ | $CF_3$ | 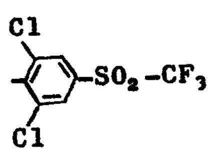 | 146 |
| 22 | $CF_3$ | $NO_2$ | 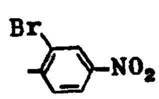 | 105 |
| 23 | $CF_3$ | $NO_2$ | 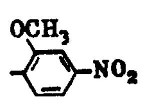 | 178 |
| 24 | $CF_3$ | $NO_2$ | 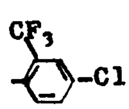 | 99 |

| | | | | |
|---|---|---|---|---|
| 25 | $CF_3$ | $NO_2$ | 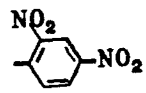 | 149 |
| 26 | $CF_3$ | $NO_2$ | 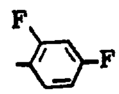 | 60 |
| 27 | $CF_3$ | $NO_2$ | 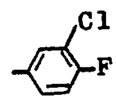 | (Oel) |
| 28 | $CF_3$ | $NO_2$ | 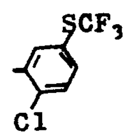 | (Oel) |
| 29 | $NO_2$ | $CF_3$ | 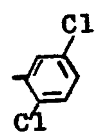 | 85 - 86 |
| 30 | $NO_2$ | $CF_3$ | 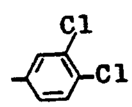 | 98 - 100 |
| 31 | $NO_2$ | $CF_3$ | 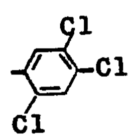 | 99 - 100 |
| 32 | $NO_2$ | $CF_3$ | 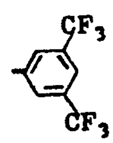 | 145 - 146 |
| 33 | $NO_2$ | $CF_3$ | 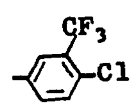 | 78 |
| 34 | $NO_2$ | $CF_3$ | 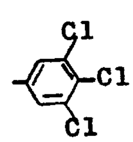 | 90 |

| | | | | |
|---|---|---|---|---|
| 35 | $NO_2$ | $CF_3$ | 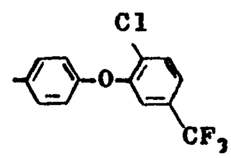 | 60 |
| 36 | $NO_2$ | $CF_3$ | 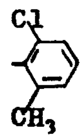 | 113 |
| 37 | $NO_2$ | $CF_3$ | 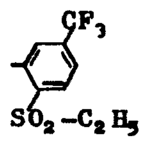 | 115 |
| 38 | $NO_2$ | $CF_3$ | 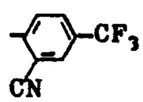 | 105 |
| 39 | $NO_2$ | $CF_3$ | 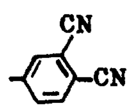 | 203 |
| 40 | $NO_2$ | $CF_3$ | 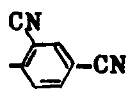 | 170 |
| 41 | $NO_2$ | $CF_3$ | 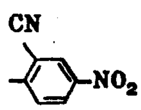 | (Oel) |
| 42 | $NO_2$ | $CF_3$ | 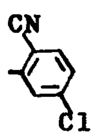 | 140 |
| 43 | $NO_2$ | $CF_3$ | 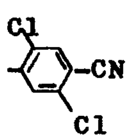 | 225 |

| | | | | |
|---|---|---|---|---|
| 44 | $NO_2$ | $CF_3$ | 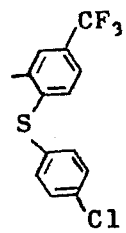 | 135 |
| 45 | $CF_3$ | $NO_2$ | 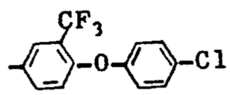 | (Oel) |
| 46 | $NO_2$ | $CF_3$ | 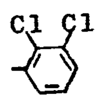 | 60 |
| 47 | $NO_2$ | $CF_3$ | 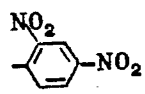 | 119 |
| 48 | $CF_3$ | $NO_2$ | 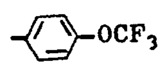 | 70 |

Patentansprüche

1. Diarylamine der allgemeinen Formel I

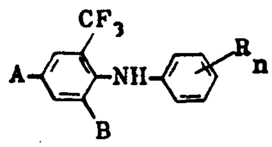

(I)

in welcher

A und B voneinander verschieden sind und für Trifluormethyl oder Nitro stehen,
n für eine ganze Zahl von 1 bis 5 steht und
R für gleiche oder verschiedene Reste aus der Gruppe Wasserstoff, Halogen, Cyan, Nitro, Alkyl, Halogenalkyl, Alkenyl, Alkinyl, Alkoxy, Alkylmercapto, Alkylsulfonyl, Halogenalkoxy, Halogenalkylmercapto, Halogenalkylsulfonyl, Aryloxy, Arylmercapto steht,
wobei ortho-ständige Substituenten R gemeinsam mit den beiden angrenzenden C-Atomen des Phenylkerns einen gegebenenfalls halogensubstituierten Heterocyclus bilden können,
und einschränkend gilt, daß $R_n$ nicht 2,4,6-Trinitro, 2,6-Dinitro-4-cyan, 2,6-Dinitro-4-trifluormethyl oder 2,4-Dinitro-6-trifluormethyl bedeutet.

2. Verfahren zur Herstellung der Diarylamine der allgemeinen Formel I gemäß Anspruch 1, dadurch gekennzeichnet, daß man

a) eine Verbindung der Formel (II)

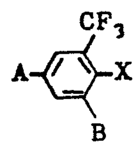 (II)

in welcher
X für Fluor, Chlor oder Brom steht und
A und B die oben angegebene Bedeutung haben, mit einem Anilin der Formel (III)

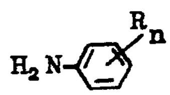 (III)

in welcher
R und n die oben angegebene Bedeutung haben,
in Gegenwart eines säurebindenden Mittels sowie gegebenenfalls in Gegenwart eines Verdünnungsmittels umsetzt, oder

b) ein Anilin der Formel (IV)

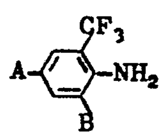 (IV)

in welcher
A und B die oben angegebene Bedeutung haben,

mit einer Verbindung der Formel (V)

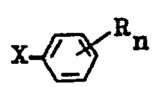 (V)

in welcher
X für Fluor, Chlor oder Brom steht und
R und n die oben angegebene Bedeutung haben,

in Gegenwart eines säurebindenden Mittels sowie gegebenenfalls in Gegenwart eines Verdünnungsmittels umsetzt.

3. Schädlingsbekämpfungsmittel, gekennzeichnet durch einen Gehalt an mindestens einem Diarylamin der allgemeinen Formel I gemäß Anspruch 1.

4. Verwendung von Diarylamine der allgemeinen Formel I gemäß Anspruch 1 zur Bekämpfung von Schädlingen.

5. Verfahren zur Bekämpfung von Schädlingen, dadurch gekennzeichnet, daß man Diarylamine der allgemeinen Formel I gemäß Anspruch 1 auf Schädlinge und/oder ihren Lebensraum einwirken läßt.

6. Verfahren zur Herstellung von Schädlingsbekämpfungsmitteln, dadurch gekennzeichnet, daß man Diarylamine der allgemeinen Formel I gemäß Anspruch 1 mit Streckmitteln und/oder oberflächenaktiven Mitteln vermischt.

0004642

Nummer der Anmeldung

Europäisches Patentamt

# EUROPÄISCHER RECHERCHENBERICHT

EP 79 10 0944

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 509 416 (I.C.I.)<br>* Ansprüche *<br>-- | 1-6 |
| A | DE - A - 2 213 081 (I.C.I.)<br>* Ansprüche *<br>-- | 1-6 |
| A | DE - A - 2 642 147 (ELI LILLEY)<br>* Ansprüche; Seite 42, Zeile 18 *<br>---- | 1-6 |

**KLASSIFIKATION DER ANMELDUNG (Int.Cl.²)**

C 07 C 87/60
C 07 D 319/08
C 07 C 93/14
121/78
147/12
149/42
A 01 N 9/20
9/12
9/14

**RECHERCHIERTE SACHGEBIETE (Int. Cl.²)**

C 07 C 87/60
147/12
121/78

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 14-05-1979 | PAUWELS |